# EUROPÄISCHE PATENTSCHRIFT

(11) **EP 2 026 228 B1**
(45) Veröffentlichungstag und Bekanntmachung des Hinweises auf die Patenterteilung: **01.08.2018**
(21) Anmeldenummer: 08075252.0
(22) Anmeldetag: 31.03.2008
(51) Int. Cl.: G06F 19/00, A61N 1/372

(54) **Fernprogrammierbares persönliches Gerät sowie Anordnung und Verfahren zum Fernprogrammieren eines persönliches Gerätes**
Remote programmable personal device and device and method for programming a personal device remotely
Appareil personnel télécommandé ainsi qu'agencement et procédé de programmation à distance d'un appareil personnel

(30) Priorität: 11.08.2007 DE 102007037947
(43) Veröffentlichungstag der Anmeldung: 18.02.2009
(73) Patentinhaber: BIOTRONIK SE & Co. KG, 12359 Berlin (DE)
(72) Erfinder: Dörr, Thomas, 12437 Berlin (DE)
(74) Vertreter: Keck, Hans-Georg

(56) Entgegenhaltungen:
- WO-A1-01/43823
- WO-A1-2006/130060
- DE-A1- 10 053 116
- US-A1- 2004 122 295
- US-A1- 2006 111 759
- US-A1- 2006 122 863

## Beschreibung

Die Erfindung betrifft ein fernprogrammierbares persönliches Gerät, insbesondere ein programmierbares implantierbares medizinisches Gerät, beispielsweise einen Herzschrittmacher, einen Defibrillator, einen Kardioverter oder dergleichen. Außerdem betrifft die Erfindung eine Anordnung für die Fernprogrammierung eines solchen persönlichen medizinischen Gerätes und ein Verfahren zum Fernprogrammieren eines programmierbaren persönlichen Gerätes.

In Zusammenhang mit Herzschrittmachern oder Defibrillatoren im Verbund mit einem Service Center ist es möglich jedoch hinlänglich bekannt, seitens eines Herzschrittmacher oder Defibrillators gewonnene, medizinische, physiologische oder Betriebsdaten zu dem zentralen Service Center zu übertragen, um diese Daten dort auszuwerten und über eine entsprechende Benutzerschnittstelle einem betreuenden Arzt zur Verfügung zu stellen.

Einige Funktionen solcher Implantate sind durch Software oder Firmware gesteuert und daher programmierbar. Hierfür besitzen die Implantate eine programmierbare Steuerung zum Steuern dieser Funktionen.

Es kommt immer wieder vor, dass nach anfänglicher Programmierung kurz vor, während oder nach der Implantation des Implantats weitere Programmierungen oder Umprogrammierungen wünschenswert sind, um das Implantat besser auf möglicherweise inzwischen veränderte Gesundheitszustände eines Patienten einstellen zu können oder um die Leistungsfähigkeit des Implantats anderweitig zu erhöhen. Häufig geschieht ein derartiges Programmieren oder Umprogrammieren dadurch, dass ein Arzt zu einem jeweiligen Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantats kann jedoch grundsätzlich auch aus der Ferne, beispielsweise über das zentrale Service Center, geschehen. Hierzu kann eine Datenverbindung zwischen dem Service Center und einem Patientengerät hergestellt werden. Das Patientengerät befindet sich üblicherweise in der Nähe des Patienten und dient quasi als Relaisstation zwischen dem Implantat und dem Service Center. Dazu besitzt das Patientengerät zwei unterschiedliche, bidirektionale Datenkommunikationsschnittstellen, auf der einen Seite eine bidirektionale Datenkommunikation mit dem Implantat und auf der anderen Seite eine bidirektionale Datenkommunikation mit dem Service Center zu ermöglichen. Die Datenkommunikationsschnittstelle für die Verbindung zwischen dem Patientengerät und dem Service Center kann dabei für eine drahtlose oder drahtgebundene Verbindung ausgebildet sein, beispielsweise über ein Mobiltelefonnetz oder über ein Festnetz. Ein solches System ist in WO 01/43823 A1 offenbart.

Während die herkömmliche Programmierung eines Implantats mit Hilfe eines Programmiergerätes durch einen Arzt in Angesicht des Patienten erfolgt, sieht der Arzt den Patienten bei der Fernprogrammierung nicht. Der Arzt hat den Patienten bei der Fernprogrammierung nicht direkt vor Augen und kann daher nicht so leicht auf unmittelbare Äußerungen des Patienten reagieren.

Die Erfinder sind zu der Erkenntnis gelangt, dass dieser Umstand bei der Gestaltung des Implantats sowie der Anordnung zum Fernprogrammieren des Implantates berücksichtigt werden sollte.

Zu diesem Zweck wird erfindungsgemäß vorgeschlagen, dass das programmierbare persönliche Gerät eine erste Datenkommunikationsschnittstelle für eine drahtlose Datenübertragung zwischen dem programmierbaren persönlichen Gerät - also im speziellen Fall dem Implantat - und einem Programmiergerät zum Programmieren des persönlichen Gerätes aus der Nähe besitzt. Darüber hinaus besitzt das programmierbare persönliche Gerät eine zweite Datenkommunikationsschnittstelle für eine drahtlose Datenübertragung, die eine Fernprogrammierung des persönlichen Gerätes erlaubt und die beispielsweise hinsichtlich der Datenübertragungstechnik oder des Datenformates von der ersten Datenkommunikationsschnittstelle verschieden ist. Beiden Datenkommunikationsschnittstellen ist gemein, dass sie es grundsätzlich erlauben, dass das programmierbare persönliche Gerät über die erste oder die zweite Datenkommunikationsschnittstelle Programmieraufträge empfangen kann. Programmieraufträge sind Datenpakete, die Steuerparameter enthalten, die über eine programmierbare Steuerung des persönlichen Gerätes die Funktionsweise des persönlichen Gerätes bestimmen. Die Summe der Steuerparameter kann auch als Programm oder als Steuerprogramm für das persönliche Gerät bezeichnet werden. Dabei kann das persönliche Gerät so eingerichtet sein, dass nur ein Teil der die Funktion bestimmenden Steuerparameter programmierbar oder durch Programmieren veränderbar ist.

Die programmierbare Steuerung ist zum Einen mit den beiden Datenkommunikationsschnittstellen wenigstens mittelbar verbunden und zum anderen mit einem Speicher verbunden, in dem mehrere Steuerparameter oder Steuerparameterkombinationen oder Steuerparameterattribute gespeichert werden können. Steuerparameterattribute sind beispielsweise Kennzeichnungen, die bezeichnen, ob ein jeweiliger Steuerparameter im Rahmen der Fernprogrammierung verändert werden kann oder nicht. Die programmierbare Steuerung ist ausgebildet, über die erste Datenkommunikationsschnittstelle für die Verbindung des persönlichen Gerätes mit dem Programmiergerät Programmieraufträge zu empfangen, die eine Vorgabe über zulässige Parameterbereiche für einen oder mehrere Steuerparameter oder Steuerparameterattribute zu einem oder mehreren Steuerparametern oder aber mehrere alternative Steuerparameterkombinationen enthalten. Ein Programmierauftrag kann auch eine Kombination dieser Steuerparameter, Steuerparameterattribute oder Steuerparameterkombinationen enthalten. Diese Art von Programmierauftrag unterscheidet sich insofern von üblichen Programmieraufträgen, als der Programmierauftrag nicht oder nicht ausschließlich Daten oder Anweisungen enthält, die zu einer direkten Veränderung der Steuerparameter und damit seiner Umprogrammierung des persönlichen Gerätes führen. Vielmehr wird das persönliche Gerät durch einen derartigen Programmierauftrag für die Fernprogrammierung eingerichtet. Hierzu ist die programmierbare Steuerung weiterhin ausgebildet, über die erste Datenkommunikationsschnittstelle empfangene Programmieraufträge, die eine solche Vorgabe zu Parameterbereichen, Parameter oder Parameterattributen enthalten, so zu verarbeiten, dass diese Parameterbereiche, die Steuerparameterattribute oder die Steuerparameterkombinationen in dem Speicher gespeichert werden. Im Falle des Empfangens eines Programmierauftrags über die zweite Datenkommunikationsschnittstelle prüft die programmierbare Steuerung, ob die in diesem Programmierauftrag enthaltenen Steuerparameter, Steuerparameterkombinationen mit den im Speicher gespeicherten Parameterbereichen oder Steuerparameterkombinationen kompatibel sind und führt einen über die zweite Datenkommunikationsschnittstelle (die Datenkommunikationsschnittstelle für die Fernprogrammierung) empfangenen Programmierauftrag nur aus, wenn dies der Fall ist. Ausführen eines derartigen Programmierauftrages bedeutet, dass die in dem über die zweite Datenkommunikationsschnittstelle empfangenen Programmierauftrag enthaltenen Steuerparameter so in dem persönlichen Gerät gespeichert werden, dass sie nach Ausführen des Programmierauftrags die Steuerung der Funktionen des persönlichen Gerätes bestimmen. Das persönliche Gerät kann einen derartigen Programmierauftrag auch über die erste Datenkommunikationsschnittstelle empfangen, wobei in jenem Fall dann keine Prüfung erfolgt, ob die in dem Programmierauftrag enthaltenen Steuerparameterkombinationen oder Steuerparameter mit den in dem Speicher gespeicherten Steuerparameter oder Steuerparameterkombinationen kompatibel sind.

Der Speicher enthält somit neben den die Funktion der programmierbaren Steuerung bestimmenden Steuerparametern weitere Informationen, nämlich Parameterbereiche, Steuerparameterkombinationen oder Steuerparameterattribute, die definieren, ob und wie ein jeweiliger Steuerparameter im Rahmen der Fernprogrammierung programmiert oder umprogrammiert werden kann. Ein Steuerparameterattribut kann beispielsweise so gesetzt sein, dass eine Umprogrammierung des entsprechenden Steuerparameters auf dem Wege der Fernprogrammierung - also durch einen über die zweite Datenkommunikationsschnittstelle empfangen Programmierauftrag - nicht möglich ist.

Das programmierbare Gerät und insbesondere dessen programmierbare Steuerung können auch dazu ausgebildet sein, verschiedene mögliche Steuerparameterkombinationen neben der jeweils gerade angewandten Parametersteuerkombination in dem Speicher zu speichern. Diese alternativen Steuerparameterkombinationen können dann über einen Programmierauftrag aktiviert werden. Hierzu kann solchen Steuerparameterkombinationen jeweils eine Kombinationskennung zugeordnet sein, so dass der Programmierauftrag nicht explizit alle Steuerparameter der Steuerparameterkombination enthalten muss, sondern vielmehr nur eine jeweilige Kombinationskennung als Verweis auf die zukünftig anzuwendende Steuerparameterkombination enthalten.

In diesem Zusammenhang ist es vorteilhaft, wenn die programmierbare Steuerung dazu ausgebildet ist, die in dem Speicher gespeicherten Parameterbereiche, Steuerparameterattribute oder Steuerparameterkombinationen an ein externes Gerät zu versenden. Dies ist insbesondere dann sinnvoll, wenn einer jeweiligen Steuerparameterkombination auch eine Kombinationskennung zugeordnet ist. Dies versetzt ein externes Gerät wie beispielsweise das Programmiergerät und den Arzt, der ein derartiges externes Gerät benutzt, dazu in die Lage, gezielt solche Steuerparameterkombinationen aufzurufen oder für die Fernprogrammierung vorgegebene, zulässige Parameterbereiche einzuhalten, wie sie im Speicher des persönlichen Gerätes gespeichert sind.

In Bezug auf die Prüfung eines über die zweite Datenkommunikationsschnittstelle empfangenen (Fern-) Programmierauftrags bieten sich zwei gleichermaßen je nach Anwendungsfall vorteilhafte Alternativen.

Zum einen kann die programmierbare Steuerung ausgebildet sein, einen über die zweite Datenkommunikationsschnittstelle empfangenen Programmierauftrag nur dann auszuführen, wenn alle in ihm enthaltenen Steuerparameter für die Fernprogrammierung zugelassen sind beziehungsweise in einem für die Fernprogrammierung zulässigen Parameterbereich liegen, sowie es im Speicher des persönlichen Gerätes gespeichert ist. Sobald nur ein in dem Programmierauftrag enthaltender Steuerparameter entweder per Steuerparameterattribut nicht für die Fernprogrammierung zugelassen ist oder außerhalb eines für die Fernprogrammierung zulässigen Parameterbereichs liegt, wird der gesamte Programmierauftrag nicht ausgeführt. Auf diese Weise kann vermieden werden, dass die in der Regel sorgfältig aufeinander abgestimmten Steuerparameter in ihrer nach Ausführen des Programmierauftrags wirksamen Kombination nicht konsistent sind.

Alternativ kann die programmierbare Steuerung auch so ausgebildet sein, dass sie einen über die zweite Datenkommunikationsschnittstelle empfangenen Programmierauftrag nur teilweise ausführt, wenn ein oder mehrere der in dem Programmierauftrag enthaltenen Steuerparameter außerhalb der für die Fernprogrammierung zugelassenen Steuerparameterbereich liegt oder per Steuerparameterattribut nicht für die Fernprogrammierung zugelassen ist.

Alternativ kann die programmierbare Steuerung auch ausgebildet sein, die beiden Alternativen in dem Sinne zu kombinieren, dass einige wesentliche Steuerparameter nur in Kombination miteinander umprogrammiert werden können, während andere Steuerparameter auch einzeln umprogrammiert werden können.

Wie bereits erläutert, ist das persönliche Gerät vorzugsweise ein aktives medizinisches Implantat, insbesondere ein implantierbarer Herzschrittmacher oder ein implantierbarer Defibrillator/Kardioverter. In jenem Fall ist die zweite Datenkommunikationsschnittstelle für die Fernprogrammierung des persönlichen Gerätes vorzugsweise eine Schnittstelle für eine drahtlose Datenkommunikation mit einer Reichweite im Bereich bis zu 5 m und entspricht insbesondere der Medical Implant Communications Service Spezifikation (MICS).

Erfindungsgemäß wird das eingangs genannte Ziel auch mit einer Anordnung zum Fernprogrammieren eines programmierbaren persönlichen Gerätes erreicht, die neben einem persönlichen Gerät der vorbeschriebenen Art auch ein Service Center zum Programmieren des persönlichen Gerätes aus der Ferne aufweist. Das Service Center besitzt wenigstens eine Datenkommunikationsschnittstelle zum wenigstens mittelbaren Verbinden des Service Centers mit dem persönlichen Gerät. Darüber hinaus besitzt das Service Center, der ausgebildet ist, wenigstens Parameterbereiche, Steuerparameterattribute oder Steuerparameterkombinationen für die programmierbare Steuerung des persönlichen programmierbaren Gerätes zu speichern. Außerdem besitzt das Service Center eine Programmiereinheit zum Programmieren des persönlichen Gerätes aus der Ferne, die ausgebildet eine Benutzeroberfläche für das Fernprogrammieren des persönlichen Gerätes zu generieren und auf diese für das Zusammenstellen eines Programmierauftrags für das persönliche programmierbare Gerät die in dem Speicher des Service Centers für dieses persönliche Gerät gespeicherten Parameterbereiche oder Steuerparameterkombinationen darzustellen.

Vorzugsweise ist das Service Center weiterhin ausgebildet, Steuerparameterattribute, Steuerparameterbereiche oder Steuerparameterkombinationen enthaltene Datenpakete seitens des programmierbaren persönlichen Gerätes zu empfangen und in dem Speicher zu speichern.

Die Programmiereinheit ist vorzugsweise so ausgebildet, dass sie nur das Zusammenstellen solcher Programmieraufträge zulässt, die mit den im Speicher des Service Centers gespeicherten Parameterbereichen oder Steuerparameterkombinationen kompatible Steuerparameter enthalten. Auf diese Weise wird ein Arzt bereits beim Zusammenstellen eines Programmierauftrags daran gehindert, einen solchen Programmierauftrag zusammenzustellen, der von dem Implantat (also dem persönlichen Gerät) nicht ausgeführt werden kann. Hier ist anzumerken, dass die Programmiereinheit üblicherweise von einer zentralen Steuereinheit (CPU) und einem im Speicher des Service Centers gespeicherten Programm gebildet sein wird.

Im Zusammenhang mit der Ausführungsvariante, bei der in dem Speicher des persönlichen Gerätes bereits aufeinander abgestimmte Steuerparameter zu Steuerparameterkombinationen zusammengefasst gespeichert und durch eine Kombinationskennung bezeichnet sind, ist es vorteilhaft, wenn die Programmiereinheit des Service Centers dazu ausgebildet ist, auf der für das Fernprogrammieren des persönlichen Gerätes generierten Benutzeroberfläche die bereits gespeicherten Steuerparameterkombinationen anzuzeigen. Ein Arzt kann dann eine der angezeigten Steuerparameterkombinationen auswählen und auf diese Weise den Programmierauftrag zusammenstellen. Die Programmiereinheit ist dann weiter dazu ausgebildet, einem entsprechenden Programmierauftrag jene Kombinationskennung hinzuzufügen, die die von einem Arzt ausgewählte Steuerparameterkombination kennzeichnet. Diese erlaubt eine besonders einfache und effiziente Programmierung des persönlichen Gerätes.

Das eingangs genannte Ziel wird außerdem durch ein Verfahren zum Fernprogrammieren eines programmierbaren persönlichen Gerätes erreicht. Das Verfahren umfasst wenigstens die Schritte
- Speichern von programmierbaren Steuerparametern zugeordneten Steuerparameterattributen in dem persönlichen Gerät
- Abfragen gespeicherte Steuerparameterattribute während einer Verarbeitung eines Programmierauftrags durch das persönliche Gerät, und
- Speichern in einem Programmierauftrag enthaltener Steuerparameter, falls diese durch die Steuerparameterattribute als durch den jeweiligen Programmierauftrag veränderbar gekennzeichnet sind.

Alternativ oder zusätzlich kann das Verfahren die folgenden Verfahrensschritte aufweisen:
- Speichern von einer oder mehreren Steuerparameterkombinationen und jeweils einer eindeutig einer jeweiligen Steuerparameterkombination zugeordneten Kombinationskennung in dem persönlichen Gerät, und
- Programmieren des persönlichen Gerätes mit der durch eine mit einem Programmierauftrag empfangenen Kombinationskennung bezeichneten Steuerparameterkombination nach Empfang des Programmierauftrags.

Die Erfindung soll nun anhand von Ausführungsbeispielen mit Bezug auf die Figuren näher erläutert werden. Von den Figuren zeigt:
- Fig. 1:: ein Anordnung zum Fernprogrammieren eines persönlichen Gerätes in Form eines Implantats; und
- Fig. 2:: ein erfindungsgemäßes fernprogrammierbares persönliches Gerät.

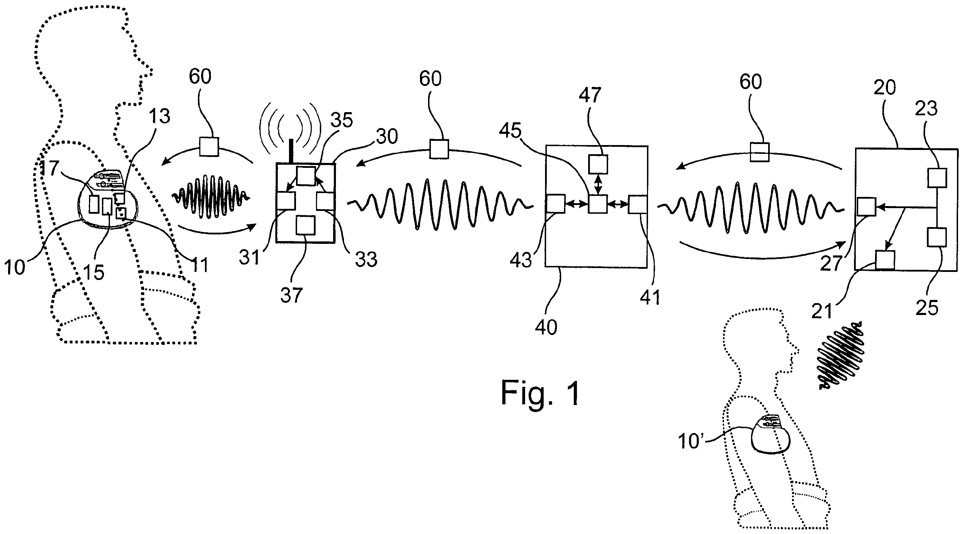

Die Anordnung zum Fernprogrammieren eines persönlichen Gerätes 10 in Form eines Implantats umfasst neben dem Implantat 10 ein Programmiergerät 20, mit dem das Implantat 10 direkt programmiert werden kann. Dies ist in Fig. 1 auf der rechten Seite durch die Darstellung des Implantats 10' in der Nähe des Programmiergerätes 20 dargestellt.

Außerdem umfasst die Anordnung zum Fernprogrammieren des Implantats 10 ein Patientengerät 30, und ein Service Center 40.

Wie sich aus Fig. 1 ergibt, kann das Programmiergerät 20 auch zur Fernprogrammierung des Implantats 10 genutzt werden, in dem das Programmiergerät 20 hierzu mit einem Service Center 40 verbunden wird.

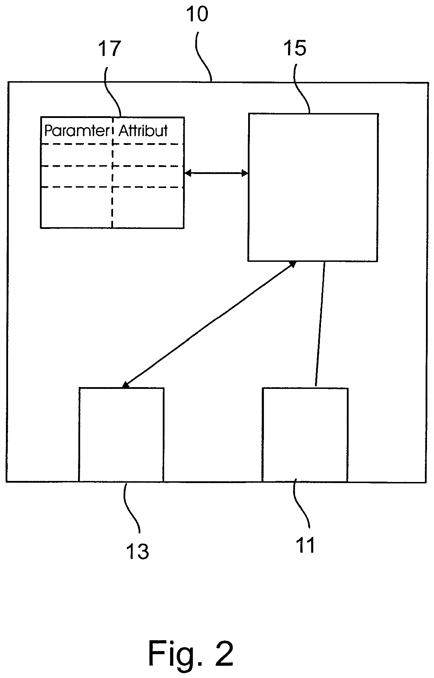

Das Implantat 10 (siehe auch Fig. 2) besitzt eines erste Datenkommunikationsschnittstelle 11, die eine drahtlose Datenkommunikation mit dem Programmiergerät 20 erlaubt. Außerdem besitzt das Implantat 10 eine zweite Datenkommunikationsschnittstelle 13, die eine drahtlose Datenkommunikation mit dem Patientengerät 30 erlaubt und die auf einer anderen Funktechnik beruht, als die Datenkommunikationsschnittstelle 11. Beide Datenkommunikationsschnittstelle 11 und 13 sind mit einer programmierbaren Steuerung 15 des Implantats verbunden, die wiederum mit einem Speicher 17 verbunden ist.

Das Patientengerät 30 besitzt ebenfalls eine erste Datenkommunikationsschnittstelle 31 für einen drahtlosen Datenaustausch mit dem Implantat 10 über dessen zweite Datenkommunikationsschnittstelle 13. Außerdem weist auch das Patientengerät 30 eine zweite Datenkommunikationsschnittstelle 33 auf, die dazu ausgebildet ist, eine Datenverbindung mit dem Server 40 herzustellen. Die erste und die zweite Datenkommunikationsschnittstelle 31 und 33 des Patientengerätes 30 sind wenigstens mittelbar über eine Steuereinheit 35 des Patientengerätes 30 miteinander verbunden. Außerdem weist das Patientengerät 30 eine eigenen Speicher 37 auf, in dem Daten zwischengespeichert werden können falls diese beispielsweise seitens des Service Centers 40 empfangen wurden und noch nicht in das Implantat 10 weiter übertragen werden konnten.

Das Service Center 40 besitzt ebenfalls eine erste Datenkommunikationsschnittstelle 43, welche der Datenkommunikation mit dem Patienten 30 dient. Die Datenverbindung zwischen der zweiten Datenkommunikationsschnittstelle 33 des Patientengerätes 30 und der ersten Datenkommunikationsschnittstelle 43 des Service Centers 40 kann dabei drahtgebunden, beispielsweise über das Telefonfestnetz erfolgen.

Auch das Service Center 40 besitzt eine Steuereinheit 45 sowie einen mit diesem verbundenen Speicher 47. Die Steuereinheit 45 und die Speicher 47 stellen in Kombination miteinander unter anderem eine Programmiereinheit für das Fernprogrammieren des Implantats 10 dar. Insbesondere sind die Steuereinheit 45 und der Speicher 47 des Service Centers 40 so ausgebildet, dass sie eine Benutzeroberfläche für das Fernprogrammieren des Implantats 10 genieren können, die es einem Benutzer erlaubt, einen Programmierauftrag für das Implantat zusammenzustellen. Hierzu kann das Service Center 40 beispielsweise über das Internet mit einem Computer eines Arztes verbunden sein, auf dessen Bildschirmoberfläche dann die Benutzeroberfläche für das Fernprogrammieren des Implantats dargestellt wird.

Wie insbesondere Fig. 2 zu entnehmen ist, ist der Speicher des Implantats 17 in Kombination mit der Steuerung 15 so organisiert, dass er neben aktuell für die Steuerung des Implantats benötigten Parametern auch diesen Parametern zugeordnete Steuerparameterattribute und gegebenenfalls auch Steuerparameterbereiche speichert. Die in dem Speicher 17 gespeicherten Steuerparameter selbst bestimmen dabei die Funktionsweise der Steuerung 15 und damit des Implantats 10. Die Steuerparameterattribute sowie die Steuerparameterbereiche bestimmen das Verhalten des Implantats bei einer Fernprogrammierung, bei der das Implantat 10 einen Programmierauftrag 60 über die zweite Datenkommunikationsschnittstelle 13 empfängt.

Die in dem Speicher 17 gespeicherten Steuerparameterattribute und Steuerparameterbereiche können nur bei einer direkten Datenverbindung zwischen dem Implantat 10' und dem Programmiergerät 20 verändert werden, sowie dies in Fig. 1 rechts unten dargestellt ist. Auf dem Wege der Fernprogrammierung können die Steuerparameterattribute und Steuerparameterbereiche nicht verändert werden.

Die Steuerung 15 des Implantats 10 ist so ausgebildet, dass sie eine Veränderung der in dem Speicher 17 gespeicherten Steuerparameter mit Hilfe eines Programmierauftrags 60 nur erlaubt, wenn das zu dem jeweiligen Steuerparameter gehörende Steuerparameterattribut so gesetzt ist, dass es eine Fernprogrammierung erlaubt. Im einfachsten Falle ist das Steuerparameterattribut ein einziges Bit, dass entweder gesetzt ist und dann eine Fernprogrammierung über die Datenkommunikationsschnittstelle 13 erlaubt; oder das Bit ist nicht gesetzt und eine Fernprogrammierung dieses Steuerparameters über die zweite Datenkommunikationsschnittstelle 13 ist nicht möglich.

Im gleichen Sinne ist die programmierbare Steuerung 15 des Implantats 10 so ausgebildet, dass sie eine Veränderung der Steuerparameter im Rahmen der Fernprogrammierung nur innerhalb des Parameterbereiches erlaubt, der in dem Speicher 17 gespeichert ist.

Um eine sinnvolle Fernprogrammierung des Implantats 10 zu erlauben, ist die Steuerung 15 des Implantats in Verbindung mit dem Speicher 17 ausgebildet, die jeweils in dem Speicher 17 gespeicherten Steuerparameter, Steuerparameterattribute und Steuerparameterbereiche über die zweite Datenkommunikationsschnittstelle 13 bis zu dem Service Center 40 zu übertragen. Dort werden diese Werte in dem Speicher 47 des Service Centers gespeichert und seitens der Steuereinheit 45 des Service Centers für das Generieren einer Benutzeroberfläche für die Fernprogrammierung herangezogen. Auf der Benutzeroberfläche werden die für die Fernprogrammierung freigegebenen Steuerparameter und Steuerparameterbereiche angezeigt. Dies geschieht vorzugsweise in Form einer graphischen Benutzeroberfläche, die über ein graphisches Eingabemittel wie beispielsweise eine Maus zu bedienen ist. Ein Arzt kann dann innerhalb der angezeigten Parameterbereiche die gewünschten Parameter anklicken und auf diese Weise einen Programmierauftrag 60 für das Implantat 10 zusammenstellen.

Da ein Service Center 40 üblicherweise mehrere Implantate versorgt, sind in dem Speicher 47 des Service Centers 40 die entsprechenden Steuerparameterattribute und Steuerparameterbereiche für die verschiedenen Implantate jeweils separat abgelegt. Vor dem Zusammenstellen eines Programmierauftrags für ein jeweiliges Implantat muss zunächst das gewünschte Implantat ausgewählt werden, damit dann die für dieses Implantat geltenden Steuerparameterattribute und Steuerparameterbereiche angezeigt werden können.

Zusätzlich oder alternativ können im Speicher 17 des Implantats 10 auch Steuerparameterkombinationen abgespeichert sein, die eine Kombination voneinander zugeordneten Steuerparametern repräsentieren. Zu jeder Steuerparameterkombination ist in dem Speicher 17 eine eindeutig der jeweiligen Steuerparameterkombination zugeordnete Kombinationskennung abgespeichert.

Auch diese Werte - Steuerparameterkombination und Kombinationskennung - überträgt das Implantat 10 bei Gelegenheit in das Service Center 40.

Bei dieser Ausführungsvariante ist das Service Center 40 - genauer gesagt dessen Steuereinheit 45 in Kombination mit dessen Speicher 47 - dazu ausgebildet, in dem Speicher 17 des Implantats 10 gespeicherte Steuerparameterkombinationen auf der generierten graphischen Benutzeroberfläche anzuzeigen. Ein Arzt kann dann eine jeweils interessante Steuerparameterkombination durch Anklicken auswählen. Die Steuereinheit 45 des Service Centers 40 generiert daraufhin einen Programmierauftrag, der die der ausgewählten Steuerparameterkombination zugeordnete Kombinationskennung enthält und überträgt diesen Programmierauftrag an das Implantats 10.

In dem Implantat 10 bilden die programmierbare Steuerung 15 und der Speicher 17 einen Programmselektor, der anhand der empfangenen Kombinationskennung die entsprechende, in dem Speicher 17 des Implantats 10 gespeicherte Steuerparameterkombination so an die entsprechenden Stellen des Speichers 17 überträgt, dass die zugehörigen Steuerparameter fortan die Funktionsweise des Implantats 10 bestimmen.

## Patentansprüche

1. Programmierbares implantierbares persönliches Gerät (10), wie ein Herzschrittmacher, Kardioverter, Defibrillator oder dergleichen, mit den Komponenten:
erste Datenkommunikationsschnittstelle (11) für eine drahtlose Datenübertragung, die so ausgebildet ist, dass das persönliche Gerät über die erste Datenkommunikations-Schnittstelle Programmieraufträge enthaltende Daten empfangen kann,
zweite Datenkommunikationsschnittstelle (13) für eine drahtlose Datenübertragung, die so ausgebildet ist, dass das persönliche Gerät auch über die zweite Datenkommunikationsschnittstelle Programmieraufträge enthaltende Daten empfangen kann, wobei die erste und die zweite Datenkommunikationsschnittstelle hinsichtlich des für die Datenübertragung erforderlichen Datenformats oder hinsichtlich der Datenübertragungstechnik oder des Datenformats unterschiedlich sind,
Speicher (17), der ausgebildet ist, wenigstens mehrere Steuerparameter und/oder Steuerparameterattribute zu speichern und programmierbare Steuerung (15) für ein Steuern von Funktionen des persönlichen Gerätes anhand von Steuerparametern, die mit der ersten und der zweiten Datenkommunikationsschnittstelle wenigstens mittelbar verbunden ist,
**dadurch gekennzeichnet dass**
die programmierbare Steuerung (15) ausgebildet ist, über die erste Datenkommunikationsschnittstelle (11) Programmieraufträge zu empfangen, die eine Vorgabe über zulässige Parameterbereiche für einen oder mehrere Steuerparameter oder Steuerparameterattribute zu einem oder mehreren Steuerparametern oder mehrere alternative Steuerparameterkombinationen enthalten,
die empfangenen Parameterbereiche, die empfangenen Steuerparameterattribute oder die empfangenen Steuerparameterkombinationen in dem Speicher zu speichern,
und in Falle des Empfangs eines Programmierauftrags über die zweite Datenkommunikationsschnittstelle (13) zu prüfen, ob in diesem Programmierauftrag enthaltene Steuerparameter oder Steuerparameterkombinationen mit den in dem Speicher gespeicherten Parameterbereichen, Steuerparameterattributen oder Steuerparameterkombinationen kompatibel sind und den über die die zweite Datenkommunikations-Schnittstelle empfangenen Programmierauftrag nur auszuführen, wenn dies der Fall ist und
in dem Speicher gespeicherte Parameterbereiche, Steuerparameterattribute oder Steuerparameterkombinationen an ein externes Gerät (30, 40; 20) zu versenden.

2. Programmierbares persönliches Gerät gemäß Anspruch 1, **dadurch gekennzeichnet, dass** die programmierbare Steuerung (15) dazu ausgebildet ist, zu einer jeweiligen Steuerparameterkombination eine diese Steuerparameterkombination kennzeichnende Kombinationskennung in dem Speicher (17) zu speichern und über die zweite Datenkommunikations-Schnittstelle an ein externes Gerät zu versenden sowie
über die zweite Datenkommunikationsschnittstelle (13) empfangene Programmieraufträge, die eine Kombinationskennung enthalten, derart auszuführen, dass die durch die jeweilige Kombinationskennung bezeichnete Steuerparameterkombination für die weitere Steuerung der Funktion des persönlichen Gerätes angewandt wird.

3. Programmierbares persönliches Gerät gemäß Anspruch 1 oder 2, **dadurch gekennzeichnet, dass** die programmierbare Steuerung (15) dazu ausgebildet ist, zu jedem Steuerparameter ein Steuerparameterattribut zu speichern, welches kennzeichnet, ob der jeweilige Steuerparameter im Rahmen des Ausführens eines über die zweite Datenkommunikations-Schnittstelle empfangenen Programmierauftrags geändert werden darf.

4. Programmierbares persönliches Gerät gemäß Anspruch 3, **dadurch gekennzeichnet, dass** die programmierbare Steuerung (15) dazu ausgebildet ist, nach Empfang eines Programmierauftrags über die zweite Datenkommunikationsschnittstelle (13) für einen jeweiligen gemäß dieses Programmierauftrags zu ändernden Steuerparameter das gespeicherte, zugehörige Steuerparameterattribut abzufragen und diesen Steuerparameter nur zu ändern, falls das zugehörige Steuerparameterattribut den Steuerparameter für eine Fernprogrammierung freigeben kennzeichnet.

5. Programmierbares persönliches Gerät gemäß Anspruch 3, **dadurch gekennzeichnet, dass** die programmierbare Steuerung dazu ausgebildet ist, nach Empfang eines Programmierauftrags über die zweite Datenkommunikationsschnittstelle (13) für alle gemäß dieses Programmierauftrags zu ändernden Steuerparameter die gespeicherten, zugehörige Steuerparameterattribut abzufragen und den Programmierauftrag nur auszuführen, falls für alle den gemäß des Programmierauftrags zu ändernden Steuerparameter das jeweils zugehörige Steuerparameterattribut die Steuerparameter für eine Fernprogrammierung freigeben kennzeichnet.

6. Programmierbares persönliches Gerät gemäß einem der Ansprüche 1 bis 5, **dadurch gekennzeichnet, dass** die programmierbare Steuerung (15) und der Speicher (17) derart zusammenwirken, dass in dem Speicher (17) die für die Steuerung aktuell verwendeten Steuerparameter und diesen zugeordnet Steuerparameterattribute, für die Fernprogrammierung freigegebene Parameterbereiche oder Steuerparameterkombinationen nebst Kombinationskennung gespeichert sind.

7. Programmierbares persönliches Gerät gemäß Anspruch 6, **dadurch gekennzeichnet, dass** die programmierbare Steuerung (15) und der Speicher (17) derart zusammenwirken, dass in dem Speicher (17) die für die Steuerung aktuell verwendeten Steuerparameter und diesen zugeordnet Steuerparameterattribute, und für die Fernprogrammierung freigegebene Parameterbereiche gespeichert sind.

8. Programmierbares persönliches Gerät gemäß Anspruch 6, **dadurch gekennzeichnet, dass** die programmierbare Steuerung (15) und der Speicher (17) derart zusammenwirken, dass in dem Speicher (17) die für die Steuerung aktuell verwendeten Steuerparameter und mögliche Steuerparameterkombinationen sowie diese jeweils eindeutig kennzeichnende Kombinationskennungen gespeichert sind.

9. Programmierbares persönliches Gerät gemäß einem der Ansprüche 1 bis 8, **dadurch gekennzeichnet, dass** das persönliche Gerät (10) ein aktives medizinisches Implantat ist.

10. Programmierbares persönliches Gerät gemäß einem der Ansprüche 1 bis 9, **dadurch gekennzeichnet, dass** das persönliche Gerät (10) ein implantierbarer Herzschrittmacher oder Defibrillator/Kardioverter ist.

11. Programmierbares persönliches Gerät gemäß einem der Ansprüche 1 bis 10, **dadurch gekennzeichnet, dass** die zweite Datenkommunikationsschnittstelle (13) des persönlichen Gerätes für eine drahtlose Kommunikation mit einer Reichweite im Bereich bis zu 5 m ausgebildet ist, insbesondere gemäß einer Medical Implant Communications Service-Spezifikation (MICS).

12. Anordnung zum Fernprogrammieren eines programmierbaren implantierbaren persönlichen Gerätes, wie einem Herzschrittmacher, Defibrillator oder dergleichen, mit den Komponenten:
- persönliches programmierbares Gerät (10) gemäß einem der Ansprüche 1 bis 11 und
- Service-Center (40) zur Programmierung des persönlichen Gerätes aus der Ferne,
wobei das Service-Center wenigstens
eine Datenkommunikationsschnittstelle (43) zum wenigstens mittelbaren Verbinden des Servicecenters mit dem persönlichen Gerät,
einen Speicher (47), der ausgebildet ist, wenigstens Parameterbereiche, Steuerparameterattribute oder Steuerparameterkombinationen für die programmierbare Steuerung (15) des persönlichen programmierbaren Gerätes zu speichern und
eine Programmiereinheit (45, 47) für das persönliche Gerät aufweist, die ausgebildet ist, eine Benutzeroberfläche für das Fernprogrammieren des persönlichen Gerätes (10) zu generieren und auf dieser für das Zusammenstellen eines Programmierauftrags für das persönliche programmierbare Gerät (10) die in dem Speicher (47) des Service-Centers (40) für dieses persönliche Gerät gespeicherten Parameterbereiche oder Steuerparameterkombinationen darzustellen,
**dadurch gekennzeichnet dass**
das persönliche programmierbare Gerät die in seinem Speicher gespeicherten Parameterbereiche, Steuerparameterattribute oder Steuerparameterkombinationen an das Service-Center (40) versendet.

13. Anordnung nach Anspruch 12, **dadurch gekennzeichnet, dass** die Programmiereinheit (45, 47) ausgebildet ist, nur das Zusammenstellen von solchen Programmieraufträgen zuzulassen, die mit den im Speicher (47) des Servicecenters gespeicherten Parameterbereichen oder Steuerparameterkombinationen kompatible Steuerparameter enthalten.

14. Anordnung nach Anspruch 12 oder 13, **dadurch gekennzeichnet, dass** die Programmiereinheit (45, 47) ausgebildet ist, in dem Speicher (47) des Servicecenters gespeicherte Steuerparameterkombinationen anzuzeigen und eine Auswahl einer der angezeigten Steuerparameterkombinationen durch eine Benutzereingabe zu ermöglichen und daraufhin einen entsprechenden Programmierauftrag zu generieren und an das persönliche Gerät abzusenden.

15. Anordnung nach Anspruch 14, **dadurch gekennzeichnet, dass** Programmiereinheit (45, 47) ausgebildet ist, einem nach Auswahl einer Steuerparameterkombination generierten Programmierauftrag eine Kombinationskennung hinzuzufügen, die die ausgewählte Steuerparameterkombination kennzeichnet.

## Claims

1. A programmable implantable personal device (10), such as a cardiac pacemaker, cardioverter, defibrillator or the like, comprising the following components:
a first data communication interface (11) for wireless data transmission, which interface is configured such that the personal device can receive data containing programming instructions via the first data communication interface,
a second data communication interface (13) for wireless data transmission, which interface is configured such that the personal device can also receive data containing programming instructions via the second data communication interface,
wherein the first and the second data communication interface are different in respect of the data format necessary for the data transmission or in respect of the data transmission technology or the data format,
a memory (17), which is configured to store at least a plurality of control parameters and/or control parameter attributes, and
a programmable controller (15) for controlling functions of the personal device on the basis of control parameters, which programmable controller is at least indirectly connected to the first and the second data communication interface,
**characterized in that**
the programmable controller (15) is configured
to receive programming instructions via the first data communication interface (11), which programming instructions contain a specification of admissible parameter ranges for one or more control parameters or control parameter attributes for one or more control parameters or a plurality of alternative control parameter combinations,
to store the received parameter ranges, the received control parameter attributes, or the received control parameter combinations in the memory,
and, in the event that a programming instruction is received via the second data communication interface (13), to check whether control parameters or control parameter combinations contained in this programming instruction are compatible with the parameter ranges, control parameter attributes or control parameter combinations stored in the memory and to execute the programming instruction received via the second data communication interface only if this is the case, and to send parameter ranges, control parameter attributes or control parameter combinations stored in the memory to an external device (30, 40; 20).

2. The programmable personal device according to claim 1, **characterized in that** the programmable controller (15) is configured to store in the memory (17), for a particular control parameter combination, a combination identification characterising said control parameter combination and to send said combination identification to an external device via the second data communication interface, and to execute programming instructions received via the second data communication interface (13) and containing a combination identification, in such a way that the control parameter combination designated by the particular combination identification is applied for the further control of the function of the personal device.

3. The programmable personal device according to claim 1 or 2, **characterized in that** the programmable controller (15) is configured to store a control parameter attribute for each control parameter, which control parameter attribute defines whether the particular control parameter can be modified within the scope of the execution of a programming instruction received via the second data communication interface.

4. The programmable personal device according to claim 3, **characterized in that** the programmable controller (15) is configured, following receipt of a programming instruction via the second data communication interface (13) for a particular control parameter that is to be modified in accordance with this programming instruction, to query the stored associated control parameter attribute and to modify this control parameter only if the associated control parameter attribute defines the control parameter as being approved for remote programming.

5. The programmable personal device according to claim 3, **characterized in that** the programmable controller is designed, following receipt of a programming instruction via the second data communication interface (13) for all control parameters to be modified in accordance with this programming instruction, to query the stored associated control parameter attribute and to execute the programming instruction if, for all control parameters to be modified in accordance with the programming instruction, the associated control parameter attribute characterizes the control parameter as being approved for remote programming.

6. The programmable personal device according to any one of claims 1 to 5, **characterized in that** the programmable controller (15) and the memory (17) cooperate in such a way that the control parameters currently used for the control and control parameter attributes associated therewith are stored in the memory (17) for the remote programming of approved parameter ranges or control parameter combinations together with combination identification.

7. The programmable personal device according to claim 6, **characterized in that** the programmable controller (15) and the memory (17) cooperate in such a way that the control parameters currently used for the control and control parameter attributes associated therewith, and parameter ranges approved for remote programming are stored in the memory (17).

8. The programmable personal device according to claim 6, **characterized in that** the programmable controller (15) and the memory (17) cooperate in such a way that the control parameters currently used for the control and possible control parameter combinations and combination identifications uniquely characterizing each of these are stored in the memory (17).

9. The programmable personal device according to any one of claims 1 to 8, **characterized in that** the personal device (10) is an active medical implant.

10. The programmable personal device according to any one of claims 1 to 9, **characterized in that** the personal device (10) is an implantable cardiac pacemaker or defibrillator/cardioverter.

11. The programmable personal device according to any one of claims 1 to 10, **characterized in that** the second data communication interface (13) of the personal device is configured for wireless communication with a range in the region up to 5 m, in particular in accordance with a Medical Implant Communications Service Specification (MICS).

12. An assembly for the remote programming of a programmable implantable personal device, such as a cardiac pacemaker, defibrillator or the like, said assembly comprising the following components:
- a personal programmable device (10) according to any one of claims 1 to 11, and
- a service center (40) for programming the personal device remotely,
wherein the service center comprises at least
a data communication interface (43) for at least indirectly connecting the service center to the personal device,
a memory (47), which is configured to store at least parameter ranges, control parameter attributes, or control parameter combinations for the programmable controller (15) of the personal programmable device, and
a programming unit (45, 47) for the personal device, which programming unit is configured to generate a user surface for the remote programming of the personal device (10) and to display on said user surface, for the compiling of a program instruction for the personal programmable device (10), the parameter ranges or control parameter combinations stored in the memory (47) of the service center (40) for this personal device,
**characterized in that**
the personal programmable device sends the parameter ranges, control parameter attributes, or control parameter combinations stored in its memory to the service center (40).

13. The assembly according to claim 12, **characterized in that** the programming unit (45, 47) is designed to allow only the compiling of programming instructions that contain control parameters compatible with the parameter ranges or control parameter combinations stored in the memory (47) of the service center.

14. The assembly according to claim 12 or 13, **characterized in that** the programming unit (45, 47) is configured to display the control parameter combinations stored in the memory (47) of the service center and to enable a selection of one of the displayed control parameter combinations by a user input, and thereupon to generate a corresponding program instruction and to send this to the personal device.

15. The assembly according to claim 14, **characterized in that** the programming unit (45, 47) is configured to add a combination identification characterizing the selected control parameter combination to a programming instruction generated following selection of a control parameter combination.

## Revendications

1. Appareil personnel programmable (10) implantable tel qu'un stimulateur cardiaque, un cardioverteur, un défibrillateur ou appareil similaire, avec les composants:
une première interface de communication de données (11) pour une transmission de données sans fil qui est conçue de telle manière que l'appareil personnel peut recevoir des données contenues dans des tâches de programmation obtenues par le biais de la première interface de communication de données,
une deuxième interface de communication de données (13) pour une transmission de données sans fil qui est conçue de manière à ce que l'appareil personnel peut également recevoir des données contenues dans des tâches de programmation par le biais de la deuxième interface de communication de données,
où les première et deuxième interfaces de communication de données sont différentes en ce qui concerne le format des données nécessaire à la transmission des données, ou en ce qui concerne la technique de transmission des données ou du format des données,
une mémoire (17) qui est conçue pour stocker au moins plusieurs paramètres de commande et/ou attributs de paramètres de commande, et
une commande (15) programmable pour la commande de fonctions de l'appareil personnel à l'aide de paramètres de commande, qui est reliée au moins indirectement avec les première et deuxième interfaces de communication de données,
**caractérisé en ce que**
la commande (15) programmable est conçue pour recevoir des tâches de programmation par le biais de la première interface de communication de données, qui contiennent une prescription concernant des domaines de paramètres accessibles pour un ou plusieurs paramètres de commande ou attributs de paramètres de commande relatifs à un ou plusieurs paramètres de commande ou plusieurs combinaisons de paramètres de commande alternatives,
pour stocker les domaines de paramètre reçus, les attributs de paramètres de commande reçus ou les combinaisons de paramètres de commande reçues dans la mémoire,
et, dans le cas de la réception d'un tâche de programmation par le biais de la deuxième interface de communication de données (13), pour vérifier si les paramètres de commande ou les combinaisons de paramètres de commande contenus dans cette tâche de programmation sont compatibles avec les domaines de paramètre,
les attributs de paramètres de commande ou les combinaisons de paramètres de commande stockés dans la mémoire et pour exécuter uniquement la tâche de programmation reçue par le biais de la deuxième interface de communication de données si c'est le cas, et
pour envoyer des domaines de paramètre, des attributs de paramètres de commande ou des combinaisons de paramètres de commande stockés dans la mémoire à un appareil externe (30, 40 ; 20).

2. Appareil personnel programmable selon la revendication 1, **caractérisé en ce que** la commande programmable (15) est conçue pour stocker dans la mémoire (17) une identification de combinaison caractérisant cette combinaison de paramètres de commande pour une combinaison de paramètres de commande respective et l'envoyer vers un appareil externe par le biais de la deuxième interface de communication de données, ainsi qu'exécuter
des tâches de programmation reçues par le biais de la deuxième interface de communication de données (13) qui contiennent une identification de combinaison de telle manière que la combinaison de paramètres de commande désignée par l'identification de combinaison donnée est employée pour la commande ultérieure de la fonction de l'appareil personnel.

3. Appareil personnel programmable selon la revendication 1 ou 2, **caractérisé en ce que** la commande (15) programmable est conçue pour stocker un attribut de paramètre de commande correspondant à chaque paramètre de commande, lequel précise si le paramètre de commande en question peut être modifié dans le cadre de l'exécution d'une tâche de programmation reçue par le biais de la deuxième interface de communication de données.

4. Appareil personnel programmable selon la revendication 3, **caractérisé en ce que** la commande (15) programmable est conçue pour demander, après la réception d'une tâche de programmation par le biais de la deuxième interface de communication de données (13), l'existence d'un attribut de paramètre de commande correspondant à un paramètre de commande respectif à modifier selon cette tâche de programmation stockée et pour ne modifier ce paramètre de commande que si l'attribut de paramètre de commande correspondant désigne le paramètre de commande disponible pour une programmation à distance.

5. Appareil personnel programmable selon la revendication 3, **caractérisé en ce que** la commande programmable est conçue pour demander, après la réception d'une tâche de programmation par le biais de la deuxième interface de communication de données (13), l'existence des attributs de paramètres de commande correspondants à tous les paramètres de commande à modifier selon cette tâche de programmation stockés et pour exécuter la tâche de programmation uniquement si l'attribut de paramètre de commande correspondant à chaque paramètre de commande correspondant à modifier désigne les paramètres de commande disponibles pour une programmation à distance.

6. Appareil personnel programmable selon l'une des revendications 1 à 5, **caractérisé en ce que** la commande (15) programmable et la mémoire (17) agissent conjointement de telle manière que, dans la mémoire (17), les paramètres de commande employés pour la commande en cours et les attributs de paramètres de commande leur étant associés, sont stockés à côté de l'identification de combinaison pour les domaines de paramètres ou les combinaisons de paramètres de commande libérés pour la programmation à distance.

7. Appareil personnel programmable selon la revendication 6, **caractérisé en ce que** la commande (15) programmable et la mémoire (17) agissent conjointement de telle manière que les paramètres de commande employés pour la commande en cours et les attributs de paramètres de commande leur étant associés, et les domaines de paramètres libérés pour la programmation à distance sont stockés dans la mémoire (17).

8. Appareil personnel programmable selon la revendication 6, **caractérisé en ce que** la commande (15) programmable et la mémoire (17) agissent conjointement de telle manière que les paramètres de commande employés pour la commande en cours et les combinaisons de paramètres de commande possibles, ainsi que les identifications de combinaisons les caractérisant sans équivoque sont stockés dans la mémoire (17).

9. Appareil personnel programmable selon l'une des revendications 1 à 8, **caractérisé en ce que** l'appareil personnel (10) est un implant médical actif.

10. Appareil personnel programmable selon l'une des revendications 1 à 9, **caractérisé en ce que** l'appareil personnel (10) est un stimulateur cardiaque implantable ou un défibrillateur/cardioverteur.

11. Appareil personnel programmable selon l'une des revendications 1 à 10, **caractérisé en ce que** la deuxième interface de communication de données (13) de l'appareil personnel est conçue pour une communication sans fil avec une portée dans une plage jusqu'à 5 m, en particulier, selon une spécification de service de communication d'implants médicaux (MICS).

12. Ensemble pour la programmation à distance d'un appareil personnel programmable implantable, tel qu'un stimulateur cardiaque, un défibrillateur ou appareil similaire, avec les composants :
- un appareil personnel programmable (10) selon l'une des revendications 1 à 11, et
- un centre de service (40) pour la programmation de l'appareil personnel à partir d'un endroit éloigné,
où le centre de service présente au moins
une interface de communication de données (43) pour la liaison au moins indirecte du centre de service avec l'appareil personnel,
une mémoire (47) qui est conçue pour stocker au moins des domaines de paramètres, des attributs de paramètres de commande ou des combinaisons de paramètres de commande pour la commande (15) programmable de l'appareil personnel programmable, et
une unité de programmation (45, 47) pour l'appareil personnel, qui est conçue pour générer une surface d'utilisateur pour la programmation à distance de l'appareil personnel (10) et sur laquelle représenter l'élaboration d'une tâche de programmation pour l'appareil personnel programmable (10) des domaines de paramètres ou combinaisons de paramètres de commande stockés pour cet appareil personnel dans la mémoire (47) du centre de service (40).
**caractérisé en ce que**
l'appareil personnel programmable renvoie les domaines de paramètres, les attributs de paramètres de commande ou les combinaisons de paramètres de commande stockés dans la mémoire au centre de service (40).

13. Ensemble selon la revendication 12, **caractérisé en ce que** l'unité de programmation (45, 47) est conçue pour ne permettre l'élaboration de telles tâches de programmation qui contiennent des paramètres de commande compatibles avec les domaines de paramètre ou combinaisons de paramètres de stockage stockés dans la mémoire (47) du centre de service.

14. Ensemble selon la revendication 12 ou 13, **caractérisé en ce que** l'unité de programmation (45, 47) est conçue pour indiquer des combinaisons de paramètres de stockage stockés dans la mémoire (47) du centre de service et pour permettre une sélection des combinaisons de paramètres de commande indiquées par une entrée d'utilisateur, et ensuite pour générer une tâche de programmation correspondante, et l'envoyer à l'appareil personnel.

15. Ensemble selon la revendication 14, **caractérisé en ce que** l'unité de programmation (45, 47) est conçue pour ajouter une identification de combinaison qui caractérise la combinaison de paramètres de commande choisie après la sélection d'une combinaison de paramètres de commande.
